Europäisches Patentamt

European Patent Office

Office européen des brevets

(19)

(11) **EP 1 306 769 A2**

(12) **EUROPEAN PATENT APPLICATION**

(43) Date of publication:
**02.05.2003 Bulletin 2003/18**

(51) Int Cl.[7]: **G06F 17/10**

(21) Application number: **02019114.4**

(22) Date of filing: **29.08.2002**

| | |
|---|---|
| (84) Designated Contracting States:<br>**AT BE BG CH CY CZ DE DK EE ES FI FR GB GR IE IT LI LU MC NL PT SE SK TR**<br>Designated Extension States:<br>**AL LT LV MK RO SI** | (71) Applicant: **HORIBA, LTD.**<br>**Kyoto (JP)** |
| | (72) Inventor: **Satake, Tsukasa**<br>**Minami-ku, Kyoto (JP)** |
| (30) Priority: **31.08.2001 JP 2001264815**<br>**19.09.2001 JP 2001284458** | (74) Representative: **Müller - Hoffmann & Partner**<br>**Patentanwälte,**<br>**Innere Wiener Strasse 17**<br>**81667 München (DE)** |

(54) **System for determining a greenhouse effect gas emission index**

(57)    The invention presents a greenhouse effect gas emission index converting system (2) for converting the emission index (M) of greenhouse effect gas (G) on the basis of input information by using a specified conversion formula (10a, 10b, ...), and calculating the emission index (M) of greenhouse effect gas more correctly and fairly in order to trade the emissions rights of greenhouse effect gas at high reliability. The greenhouse effect gas emission index converting system (2) of the present invention includes a data input unit (8) for entering various data for calculating the index emission (M) of greenhouse effect gas, an information input unit (9) for incorporating the conversion information (10a, 10b, ...) for calculating the emission index (M) of greenhouse effect gas by using various data, an operation processing unit (2, 10) for calculating the emission index (M) of greenhouse effect gas by calculating various data according to the conversion information (10a, 10b, ...) , and an information output unit (11) for issuing the calculated emission index (M).

F i g. 1

**Description**

**BACKGROUND OF THE INVENTION**

**Field of the Invention**

The present invention relates to a greenhouse effect gas emission index converting system for regulating the emission right or emissions rights of greenhouse effect gas on the basis of a single emission index in order to prevent global warming, and trading this emission right or emissions rights on the basis of the single emission index.

**Description of the Prior Art**

It has been hitherto feared that the global warming be promoted by plant emission gases such as $CO_2$, $CH_4$, $N_2O$, HFC, PFC, and $SF_6$ designated as greenhouse effect gases, and a general framework of emissions rights of greenhouse effect gas is agreed among nations in the world. At the present, however, means for exercising the emissions rights of greenhouse effect gas appropriately by each nation, and means for controlling the emission of greenhouse effect gas, and related penalties are in the process of discussion.

That is, any district or enterprise has no means for calculating the emission index of greenhouse effect gas effective for determining the emission framework of greenhouse effect gas, and regulations of emission amount are not determined, seriousness of effects of increasing emission of greenhouse effect gas on the global environment is anticipated. Accordingly, the districts and enterprises are debating to establish certain regulating means by determining the emission framework of greenhouse effect gas, and various calculation methods are proposed for assessing the emission index of greenhouse effect gas for this purpose.

Generally, as calculation method of emission index of greenhouse effect gas, the carbon amount is converted on the basis of the accumulated information by research institutes and industrial societies, and in the semiconductor industry, for example, the following formula (1) is proposed and employed as the Million Metric Ton Carbon Equivalent (MMTCE).

$$MMTCE = \frac{12}{44} \cdot \sum_i \{(Pi \times 0,9) \times (1-C) \times (1-A \times F) \times GWPi\} \quad \ldots \quad (1)$$

where Pi is purchase amount, C is consumption rate in the process, A is abatement or decreasing efficiency by, F is a fraction of gas volume in an abatement equipment (which may also be referred to as a decontamination apparatus in the following) or decomposition factor of abatement equipment, and GWPi is 100-year value of global warming potential for gas.

The above-mentioned purchase amount Pi is the amount of greenhouse effect gas purchased by each enterprise, the abatement efficiency A shows the efficiency guaranteed by the manufacturer of the abatement equipment attached to the process, and GWPi is determined by the Intergovernmental Panel on Climate Change (IPCC) as the environmental management office.

By suppressing the emission index of the greenhouse effect gas calculated by using the conversion formula (1), for example, within the specified range assigned for each enterprise, it is considered to define limits on total emission of greenhouse effect gas of each enterprise. That is, by determining the emission index of greenhouse effect gas, each enterprise can judge if the own total emission may settle within the emission framework or not, and, in future, an enterprise short of the emissions rights may purchase from an enterprise having an enough emissions rights.

However, the consumption rate C by the above-mentioned process or the decomposition factor F of the abatement equipment vary significantly depending on the situation of the process, but the consumption rate C is always constant in the above-mentioned formula (1). Similarly, the decline of decomposition factor F of the abatement equipment is calculated to be a constant abatemnet efficiency A, but actually fluctuations are inevitable. Accordingly, if the input values are correct, the emission index of the greenhouse effect gas determined according to the above-mentioned formula (1) was often different from the actual situation.

In addition, it was not always fair in this method of calculation of own plant emission gas by self-declaration by the manager of the enterprise. For example, if a wrong value is entered from the greenhouse effect gas purchase by the enterprise, or the efficiency of the abatement equipment is evaluated higher than the actual value, the calculation of the above-mentioned formula (1) may be significantly different from the actual emission amount. In the above-mentioned formula (1), moreover, the greenhouse effect gas as the byproduct of the process is not considered in calculation, a larger amount of greenhouse effect gas may be actually discharged by this byproduct.

Accordingly, for calculation of emission index of the above-mentioned greenhouse effect gas, various conversion formulas (Tier 1, Tier 2A, Tier 2B, Tier 2C, etc.) are proposed by IPCC in order to calculate fair values. In this case, however, it is hard to judge which conversion formula should be used to determine the emission formula as the reference of trade of emissions rights, and it may be hard to agree in actual trade of emission amount.

Constants used in conversion formulas determined by IPCC (for example, consumption rate C in process, decreasing efficiency A by abatement equipment, decomposition factor F of abatement equipment, and global warming potential for gas GWPi), and conversion formulas are not fixed but should be changed and updated along with progress in the instrument and efficiency of manufacturing line. Accordingly, every time the conversion formula or constant is reviewed, the emission index of greenhouse effect gas must be calculated newly.

## SUMMARY OF THE INVENTION

The invention is devised in the light of such background, and it is hence an object thereof to present a greenhouse effect gas emission index converting system for converting the emission index of greenhouse effect gas on the basis of input information by using a specified conversion formula, and calculating the emission index of greenhouse effect gas more correctly and fairly in order to trade the emissions rights of greenhouse effect gas at high reliability.

The invention composes the means for solving the problems as follows. That is, the greenhouse effect gas emission index converting system of the present invention includes a data input unit for entering various data for calculating the index emission of greenhouse effect gas, an information input unit for incorporating the conversion information for calculating the emission index of greenhouse effect gas by using various data, an operation processing unit for calculating the emission index of greenhouse effect gas by calculating various data according to the conversion information, and an information output unit for issuing the calculated emission index.

In the above-mentioned greenhouse effect gas emission index converting system, since the conversion information for converting into the emission index of greenhouse effect gas is incorporated through the information input unit, the conversion information can be always updated to the latest value, and the method of converting into the emission index of greenhouse effect gas can be unified on the whole. Therefore, on the basis of the emission index of greenhouse effect gas calculated according to the unified converting method, every nation, every district or every enterprise can define the emission amount of greenhouse effect gas or trade the emissions rights each other.

Various data to be entered through the data input unit include, for example, the number of cylinders filled with greenhouse effect gas, residual gas rate in returned cylinders, gas consumption rate, type of process, decomposition factor of abatement equipment, and other data, which can be entered through the keyboard.

If a part of the above-mentioned various data includes measured concentration and flow rate of greenhouse effect gas, and when calculating the emission index by integration of their multiplied products, by using analyzers and flow meters capable of measuring the concentration and flow rate of gas emitted through flue, these measured values may be determined as various data from the integration of the emission amount of the greenhouse effect gas by actually measuring the emission index of the greenhouse effect gas.

In this case, since the emission amount and emission rate of greenhouse effect gas can be measured in real time, the values can be instantly used in trade. Since the emission index of greenhouse effect gas is also determined from the measured values by the gas analyzer and flow meter, the actual emission amount of greenhouse effect gas can be determined more accurately. Fluctuations of emission index of greenhouse effect gas caused by decline of efficiency of abatement equipment, process abnormality, or appearance of byproducts can be correctly measured, and unlike the prior art, the calculated emission index is not different from the actual value.

In particular, since the emission index of greenhouse effect gas does not vary depending on the values entered by the operators, the reliability is assured, and a fair trade can be made. Therefore, in future, the emission index of greenhouse effect gas may be expressed at MMTCE, and the emission tax can be charged.

When at least one of incorporation of various data in the above-mentioned data input unit, incorporation of conversion information in the above-mentioned information input unit, or output of emission index in the above-mentioned information output unit is executed through the network, the conversion information can be updated easily, and the conversion information may be unified more easily to define the emission amount of greenhouse effect gas, and the emissions rights can be traded on the network.

When the above-mentioned conversion information involves the conversion formula for calculating the emission index of greenhouse effect gas determined by the monitoring organization for organizing the emission amount of greenhouse effect gas and/or the constants used in such conversion formula, contents of calculation using various data in the operation processing unit can be easily changed, and when the conversion formula for calculating the emission index of greenhouse effect gas or the constants used in the conversion formula are revised by the IPCC or the like, it is readily possible to calculate by using the revised conversion formula.

The contents of the above-mentioned calculation may be offered in various methods, and may be supplied, for example, by a calculation program that can be executed by the above-mentioned operation processing unit compiled on the basis of the conversion formula disclosed by the IPCC or the like. Further, the conversion formula for converting the various input data into the emission index of greenhouse effect gas may be expressed by conforming to a specified format such as matrix calculation, and the contents of calculation may be supplied in a format of matrix.

When each greenhouse effect gas emission index converting system stores plural calculation programs supplied as conversion formulas, the conversion formulas may be properly changed over and used depending on the situation, so that the emissions rights may be traded smoothly.

When the above-mentioned conversion information has the conversion rate for calculating the emission expense of greenhouse effect gas by multiplying with the emission index of greenhouse effect gas, and the above-mentioned operation processing unit has a market reference function of entering the above-mentioned conversion rate, exchange rate and other trade information from the trade market of greenhouse effect gas through the above-mentioned information input unit, if the conversion rate varies due to trade market fluctuations, due reactions may be made in real time.

Further, when the above-mentioned operation processing unit has a market disclosure function of outputting the trade amount by multiplying the trade information such as conversion rate and exchange rate with the emission index of the above-mentioned greenhouse effect gas to the trade market of greenhouse effect gas through the above-mentioned information output unit, trade with the market can be automated, and the emissions rights of greenhouse effect gas can be traded easily.

## BRIEF DESCRIPTION OF THE DRAWINGS

**Fig. 1**    is a diagram showing a configuration of a green-house effect gas emission index converting system of the invention.

**Fig. 2**    is a diagram showing a modified example of the greenhouse effect gas emission index converting system in Fig. 1.

**Fig. 3**    is a diagram showing a different example of the greenhouse effect gas emission index converting system.

**Fig. 4**    is a diagram showing a modified example of the greenhouse effect gas emission index converting system in Fig. 3.

## DETAILED DESCRIPTION OF THE PREFERRED EMBODIMENTS

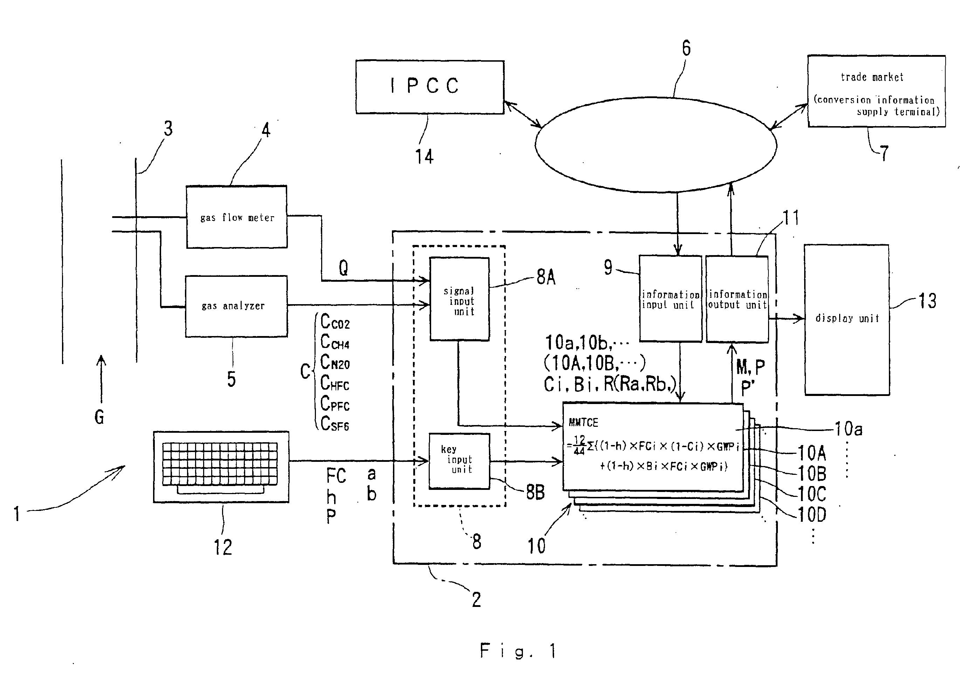

Fig. 1 is a diagram showing an example of a greenhouse effect gas emissions rights converting system 1 of the invention. In Fig. 1, reference numeral 2 is a greenhouse effect gas emission index converter (operation processing unit in this example) installed in a factory emitting greenhouse effect gas, reference numeral 3 is an emission passage of emission gas such as factory flue, reference numeral 4 is a gas flow meter installed in the emission passage 3, reference numeral 5 is a gas analyzer for analyzing the concentration of gas flowing in the emission passage 3, reference numeral 6 is the Internet as an example of network, and reference numeral 7 is an example of trade market connected to each greenhouse effect gas emission index converter 2 through the Internet 6, which also functions as a conversion information supply terminal 7 in this example as mentioned below.

The greenhouse effect gas emission index converter 2 includes a data input unit 8 for entering various data necessary for converting into the emission index of greenhouse effect gas, an information input unit 9 for entering conversion information or the like for converting into the emission index of greenhouse effect gas through the Internet 6, an operation processing unit 10 for converting by using the various data obtained through the data input unit 8 and the conversion information obtained through the information input unit 9, and information output unit 11 for sending out the emission index of greenhouse effect gas obtained in the operation processing unit 10 to the Internet 6.

The keyboard 12 is a input terminal or device of the greenhouse effect gas emission index converter 2 for entering e.g. the purchase weight FC of greenhouse effect gas, rate h of unused gas, type P of process, decomposition factor a of abatement equipment of the plant, decomposition factor d of abatement equipment of each decomposition system, and others. The display unit 13 is a monitor of the greenhouse effect gas emission index converter 2, and 14 is a monitoring organization for organizing the emission amount of greenhouse effect gas such as IPCC.

The above-mentioned emission passage or process effluent 3 is the passing route of the whole gas emitted from, for example, a semiconductor manufacturing process, and when gases emitted from plant facilities are released through several process effluents 3, a pair of gas flow meter 4 and gas analyzer 5 must be installed in each process effluent 3.

The gas flow meter 4 is available in various types, and for example, the ultrasonic type, Doppler type, and Karman's vortex type may be used.

The gas analyzer 5 is capable of measuring the concentration of greenhouse effect gas contained in the emission gas G flowing in the process effluent 3 in real time, and preferably the FTIR (Fourier transform infrared ray) gas analyzer is used. Or for batch measurement, gas chromatography or mass analyzer may be used. Components to be measured are, for example, $CO_2$, $CH_4$, $N_2O$, HFC, PFC, and $SF_6$, and in the following explanation, the concentrations of the individual greenhouse effect gases are expressed as $c_{CO_2}$, $c_{CH_4}$, $c_{N_2O}$, $c_{HFC}$, $c_{PFC}$, and $c_{SF_6}$, respectively.

Types of greenhouse effect gas are not specified in the invention, and hence types of the gas analyzer 5 are not limited. That is, any greenhouse effect gas may be measured, and the concentration of any greenhouse effect gas may be measured by any gas analyzer 5.

The above-mentioned data input unit 8 comprises a signal input unit 8A for entering measured values by the gas flow meter 4 and FTIR 5, for example, and a key input unit 8B for entering various data from the keyboard 12. The above-mentioned operation processing unit 10 has various conversion formulas 10a, 10b, ... for calculating the emission index of greenhouse effect gas by using various data $c_{CO_2}$, ..., FC, h, P, a, b, ... from the data input unit 8.

The above-mentioned conversion formulas 10a, 10b, ... (conversion information) are conversion formulas designated by the monitoring organization for organizing the emission of greenhouse effect gas such as IPCC, and are stored in format to be executed by the operation processing unit 10, for example, conversion programs 10A, 10B, ... In the following explanation of this example, the conversion programs 10A, 10B, ... to be calculated on the basis of the conversion formulas 10a, 10b, ... of emission index M of greenhouse effect gas are presented as example of conversion information, but the conversion information of the invention is not limited to the conversion programs 10A, 10B, ... For example, the operation matrix of the conversion formulas 10a, 10b, ... of emission index M of greenhouse effect gas expressed in matrix calculation can be stored as conversion information, and various modifications are possible.

At the side of the above-mentioned conversion information supply terminal 7, for example, communicating with the IPCC 14 through the Internet 6, it is designed to browse the latest conversion formulas 10a, ... for calculating the emission index of greenhouse effect gas determined by the IPCC 14, and values of coefficients used in these conversion formulas 10a, 10b, ... Conversion programs 10A, 10B, ... are compiled for executing the calculation of emission index of greenhouse effect gas on the basis of the conversion formulas 10a, 10b, ... revised by the IPCC 14, by the operation processing unit 10, and these conversion programs 10A, 10B, ... are distributed as conversion information to each greenhouse effect gas emission index converter 2 through the Internet 6.

Therefore, when each greenhouse effect gas emission index converter 2 executes a specified program among the conversion programs 10A, 10B, ... received from the conversion information supply terminal 7, the emission index of greenhouse effect gas (for example, carbon equivalent) M using a common conversion formula can be calculated, and it can be displayed in the display unit 13. Further, each greenhouse effect gas emission index converter 2 issues the emission index M of greenhouse effect gas through the information output unit 11, so that the greenhouse effect gas emissions rights can be traded with other greenhouse effect gas emission index converter. At this time, a trade market 7 may intermediate.

In the configuration of this example, the conversion formulas 10a, 10b, ... for calculating the emission index of greenhouse effect gas can be changed in batch by an instruction from the conversion information supply terminal 7, or by using an adjusted conversion formula, the emission index of greenhouse effect gas can be obtained very easily on the whole. That is, the conversion formula of emission index of greenhouse effect gas determined by the IPCC 14 or the like can be easily used in guidance of reduction of the emission amount of greenhouse effect gas or trade of emissions rights of greenhouse effect gas.

In trade of emissions rights of greenhouse effect gas, if agreed between the trading partners, needless to say, the emissions rights may be traded by using other conversion formula than the conversion formula of emission index of greenhouse effect gas specified by the IPCC 14 or the like. Distribution of conversion programs 10A, 10B, ..., or designation of conversion formula used in calculation of emission index of greenhouse effect gas is not always commanded from the trade market such as conversion information supply terminal 7, but may be also offered from others such as IPCC 14, JEITA (Japan Electronics and Information Technology Industries Association), government office (Ministry of Economy and Industry), etc.

On the other hand, as the conversion formula for calculating the emission index of greenhouse effect gas, for example, the following conversion formulas are proposed at the present. That is, the conversion formula for calculating the approximate value of the total emission amount of greenhouse effect gas (emission index of greenhouse effect gas) called Tier 1 in IPCC 14 is expressed in formula (2) below in terms of carbon equivalent (MMTCE).

$$MMTCE = \frac{12}{44} \cdot \sum_i \{(1-h) \times FCi \times (1-Ci) \times GWPi + \ldots$$
$$\ldots + (1-h) \times Bi \times FCi \times GWP_{CF_4}\}$$

$$\ldots \quad (2)$$

where FCi is i-th purchase gas weight, GWPi is 100-year value of global warming index GWP of i-th gas, h is the rate of residual gas in returned cylinders, Ci is the default value of gas consumption rate, Bi is the default value of generation rate of $CF_4$ gas as by product, and $GWP_{CF4}$ is 100-year value of global warming index GWP of $CF_4$ gas.

The 100-year value of global warming index GWP is one of conversion information determined by the IPCC 14, and at the present it is determined as shown in Table 1. If necessary to revise the value of the global warming index GWP, the latest value is distributed from the conversion information supply terminal 7, so that it may be always kept in the latest state.

| Type of gas | Value of GWP | Life (years) |
|---|---|---|
| $CF_4$ | 6500 | 50000 |
| $C_2F_6$ | 9200 | 10000 |
| $C_3F_8$ | 7000 | 2600 |
| $C_4F_8$ | 8700 | 3200 |
| $C_5F_8$ | 90 | 0,98 |
| $CHF_3$ | 11700 | 264 |
| $SF_6$ | 23900 | 3200 |
| $NF_3$ | 8000 | 700 |
| $CO_2$ | 1 | 50-200 |

Similarly, the conversion formula for calculating the approximate value of the total emission amount of greenhouse effect gas (emission index of greenhouse effect gas) called "Tier 2B", "Tier 2C" in IPCC 14 is expressed in formula (3) below in terms of carbon equivalent (MMTCE).

$$MMTCE = \frac{12}{44} \cdot \sum_i \{(1-h) \times FCi \times (1-Ci) \times (1-a_i d_i) \times GWPi + \ldots$$
$$\ldots + (1-h) \times Bi \times FCi \times (1-a_i d_{CF_4}) \times GWP_{CF_4}\}$$

$$\ldots \quad (3)$$

where $a_i$ is the decomposition factor of the abatement equipment of the plant of i-th gas, $d_i$ is the decomposition factor of the abatement equipment, $d_{CF_4}$ is the decomposition factor of $CF_4$ of the abatement equipment, and others are the same as in formula (2).

In the case of Tier 2C, default values Bi, Ci are constant regardless of type of the plant, and in the case of Tier 2B, default values Bi and Ci are different whether the plant is of dry etching or CVD.

The conversion formula for calculating the approximate value of the total emission amount of greenhouse effect gas (emission index of greenhouse effect gas) called "Tier 2A" is expressed in formula (4) below in terms of carbon equivalent (MMTCE).

$$MMTCE = \frac{12}{44} \cdot \sum_{i}\left\{(1-h)\cdot\sum_{p}\left[FCi,p \times (1-Ci,p) \times (1-a_{ip}d_{ij}) \times GWPi + \ldots\right.\right.$$
$$\left.\left. \ldots + Bi,p \times FCi,p \times (1-a_{ip}d_{CF_4,j}) \times GWP_{CF_4}\right]\right\}$$

$$\ldots \quad (4)$$

where p is the number of processes or types of process, FCi,p is i-th purchase gas weight in process p, Ci,p is the default value of consumption rate of i-th gas in process p, $a_{ip}$ is the decomposition factor of abatement equipment of enterprise or plant of i-th gas in process p, $d_{ij}$ is the decomposition factor of each decontamination system, Bi,p is the generation rate of $CF_4$ gas produced in process p, $d_{CF4j}$ is the decomposition factor of $CF_4$ of abatement equipment, and others are same as in formulas (2) and (3).

The conversion formulas shown in the above-mentioned formulas (2) to (4) are mainly calculated by using the data (FC, h, P, a, b) entered through the above-mentioned keyboard 12, but the gas consumption rate Ci and $CF_4$ gas generation rate Bi in formulas (2) and (3) are constant values (default values) used in the conversion formulas 10a, 10b, ... entered through the above-mentioned information input unit 9. Therefore, these constants Ci, Bi are adjusted properly by the judgement of the IPCC 14, and it is possible to react promptly by using the greenhouse effect gas emission index converting system 1 of the invention. Of course, these constants Ci, Bi and conversion formulas 10a, 10b, ... may be also entered through the keyboard 12. On the other hand, the gas consumption rate Ci,p, and $CF_4$ gas production rate Bi,p in formula (4) are determined from the measured values by the gas analyzer 5.

In the greenhouse effect gas emission index converting system 1 of the example, since the gas flow meter 4 and gas analyzer 5 are provided in the process effluent 3, by multiplying the measured values Q, C obtained from these instruments 4, 5, it is also possible to determine the emission amount $Q_i$ of the greenhouse effect gases $CO_2$, $CH_4$, $N_2O$, HFC, PFC, and $SF_6$ ($Q_{CO_2}$, $Q_{CH_4}$, $Q_{N_2O}$, $Q_{HFC}$, $Q_{PFC}$, and $Q_{SF_6}$) Formula (5) shows this relation.

$$Q_i = C_i \times Q \qquad (5)$$

where i denotes the type of greenhouse effect gases $CO_2$, $CH_4$, $N_2O$, HFC, PFC, and $SF_6$.

When the emission amount of greenhouse effect gases $CO_2$, $CH_4$, $N_2O$, HFC, PFC, and $SF_6$ in the above-mentioned formula (5) is multiplied by the global warming index GWP, and the sum is calculated, as shown in formula (6), the momentary value $Q_{total}$ of the total emission of the greenhouse effect gases converted to $CO_2$ is determined.

$$Q_{total} = \sum_{i}(C_i \times Q \times GWPi) \qquad \ldots \quad (6)$$

where i denotes the type of greenhouse effect gases $CO_2$, $CH_4$, $N_2O$, HFC, PFC, and $SF_6$.

The operation processing unit 2 integrates the momentary value $Q_{total}$ of emission for a specified time according to formula (7), and the emission index of greenhouse effect gas (carbon equivalent) MMTCE is determined.

$$MMTCE = \int Q_{total} dt \qquad (7)$$

That is, the greenhouse effect gas emission index converting system 1 of the invention comprises the gas flow meter 4, gas analyzer 5, and signal input unit 8A (data input unit 8) for entering measured signals C, Q, ... from them, and the emission index M of greenhouse effect gas is more accurate and a fair index is obtained. Calculation as shown in the above-mentioned formulas (5) to (7) can be executed by the conversion programs initially stored, for example, in the operation processing unit 2, but may be also executed by the conversion programs 10A, 10B, ... distributed from the above-mentioned conversion information supply terminal 7.

When calculating the emission index of greenhouse effect gas by the calculation as shown in the above-mentioned formulas (5) to (7), it is not necessary to enter the numerical data by using the keyboard 12, and not only the complicated input work can be omitted, but also appropriate countermeasures may be taken by discovering decline of efficiency of abatement equipment or occurrence of abnormality of plant in an earlier stage. In particular, the FTIR presented as an example of gas analyzer 5 is an analyzer of multiple components, and components to be measured can be set freely, and therefore a new gas designated as greenhouse effect gas by the IPCC 14 may be entered as part of the conversion information, and the emission index of greenhouse effect gas can be converted by using this information.

In the conversion formulas presented above, presenting an example of expressing the emission index M of greenhouse effect gas in terms of carbon equivalent (MMTCE), the degree of greenhouse effect is expressed adequately by using one-dimensional numerical value. However, the invention is not intended to limit this point. That is, the emission index M of greenhouse effect gas may be any index expressing the emission index of each greenhouse effect gas.

Anyway it is a feature of the greenhouse effect gas emission index converting system 1 of the invention that the emissions rights of greenhouse effect gas is calculated on the basis of a single emission index by each greenhouse effect gas emission index converter 2, by incorporating the conversion formulas (conversion information) for calculating the emission index M of greenhouse effect gas through the network 6 as shown in respective formulas (2) to (7).

As in this example, when each greenhouse effect gas emission index converter 2 stores plural conversion programs 10A, 10B, ..., if the enterprises mutually agree, it is possible to trade the emissions rights by using the emission index of greenhouse effect gas calculated according to other conversion formula different from the conversion formula determined by the general trade market 7 or IPCC 14.

In addition, by using the greenhouse effect gas emission index converting system 1 of the invention, it is also possible to determine the trade information R per unit volume of carbon dioxide (that is, conversion rate Ra or exchange rate Rb for converting the emission index into amount of money for trade) used in the greenhouse effect gas emissions rights trade in the trade market 7 or the IPCC 14. That is, as shown in formula (8) below, by multiplying the emission index M of greenhouse effect gas and the conversion rate Ra, the emission expense P of greenhouse effect gas (that is, the trade amount P') can be calculated.

$$P = M \times R \tag{8}$$

That is, the conversion result information determined by approximate calculation the operation processing unit 10 using the above-mentioned conversion formula and issued through the Internet 6 can be expressed not only by the emission index M of greenhouse effect gas (generally carbon equivalent MMTCE), but also by the emission expense P, trade amount P', or total amount of greenhouse effect gases or total amount of $CO_2$.

When trading between nations differing in currency, meanwhile, the monetary exchange rate Rb is taken into consideration as the above-mentioned trade information R, and by calculating the trade amount P', the emissions rights of greenhouse effect gas can be traded between different nations.

Incidentally, when the information output unit 11 issues the emission index M of greenhouse effect gas to the trade market 7 through the Internet 6, the trade market 7 can check how much greenhouse effect gas is emitted in each factory. That is, while the trade market 7 is adjusting the emission of greenhouse effect gases within a specified range in the entire district, an enterprise emitting an excessive amount of greenhouse effect gases can purchase the emissions rights of greenhouse effect gases from other enterprise emitting a smaller amount of greenhouse effect gases.

In the invention, the output of the emission index M of greenhouse effect gas from each enterprise is not limited to the trade market 7 alone. That is, the emission index M of greenhouse effect gas can be also issued to the government office (Ministry of Economy and Industry), JEITA or other association.

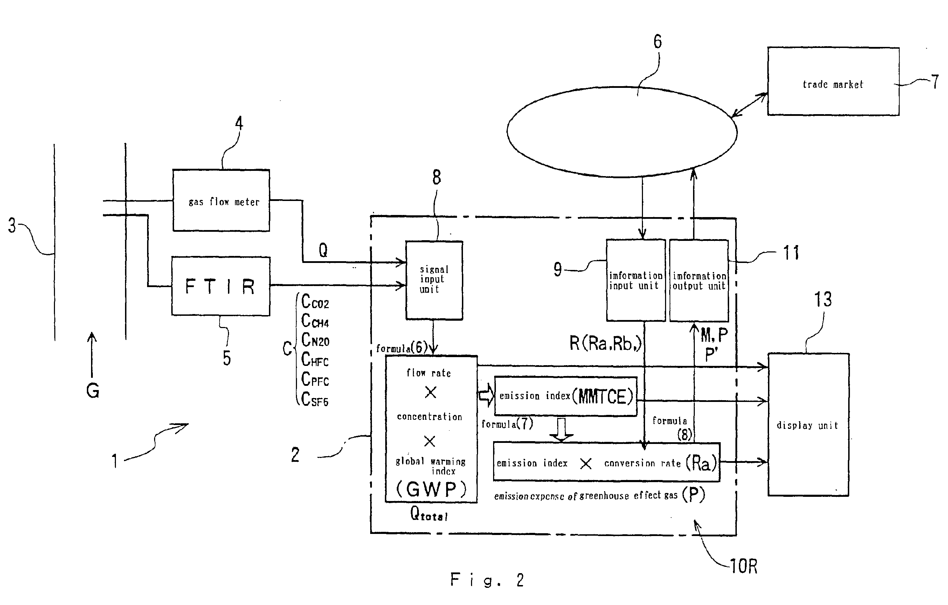

Fig. 2 is a diagram showing a modified example of the above-mentioned greenhouse effect gas emission index converting system 1, and same reference numerals as in Fig. 1 represent corresponding parts. The greenhouse effect gas emission index converting system 1 of the example is to determine the emission index of greenhouse effect gas from the measured concentration C and flow rate Q of each gas.

Therefore, the greenhouse effect gas emission index converter 2 is an operation processing unit connected to the gas flow meter 4 and gas analyzer 5, and this operation processing unit 2 comprises a signal input unit, as the data input unit 8, for entering the flow rate Q measured by the gas flow meter 4, and the concentration C of each gas measured by the gas analyzer 5, and the above-mentioned information input unit 9 enters the trade information R (for example, conversion rate Ra and exchange rate Rb), and the emission index M of greenhouse effect gas and emission expense P (including the trade amount P' in consideration of exchange rate Rb) are issued through the information output unit 11.

Therefore, in the operation processing unit 2, as shown in the drawing, an operation program 10R for processing the operation of the above-mentioned formulas (6) to (8) described above is stored, and the operation processing unit 2 executes this conversion program 10R, and thereby the emission index M of greenhouse effect gas and emission expense P are calculated, and are issued to the display unit 13 or information output unit 11.

That is, since the emission amount of greenhouse effect gas is determined by calculating from the measured values of the gas flow meter 4 and gas analyzer 5, not only the accurate emission amount of greenhouse effect gas emitted through the process effluent 3 can be measured, but also the conversion program 10R is operated to convert into the emission expense P (or trade amount P') of the greenhouse effect gas in real time, so that the emissions rights of greenhouse effect gas can be traded easily.

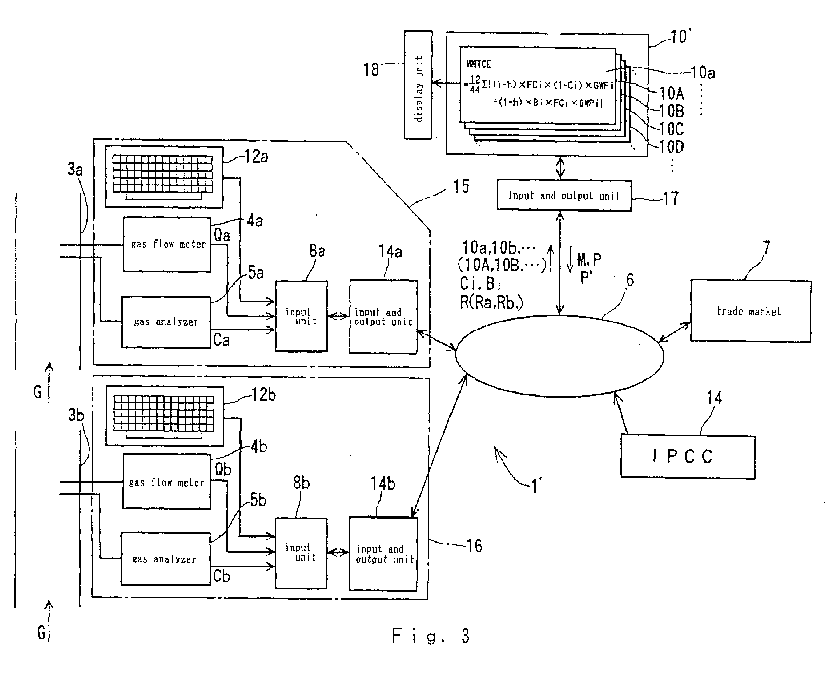

Fig. 3 shows other example of the greenhouse effect gas emission index converting system of the invention. In Fig. 3, same reference numerals as in Fig. 1 are same or corresponding parts and detailed description is omitted.

In Fig. 3, reference numeral 10' is an operation processing unit installed in each enterprise, 3a, 3b are process effluents controlled by the operation processing unit 10', reference numerals 4a, 4b are gas flow meters installed in the process effluents 3a, 3b, reference numerals 5a, 5b are gas analyzers, reference numerals 8a, 8b are input units for entering measured values Qa, Ca, Qb, Cb by the measuring instruments 4a, 4b, 5a, 5b, reference numerals 12a, 12b are keyboard, and reference numerals 14a, 14b are input and output units for connecting the signal input units 8a, 8b to the operation processing unit 2 through the Internet 6. Reference numerals 15, 16 are measuring units installed in the process effluents 3a, 3b.

Reference numeral 17 is an input and output unit (information input unit and information output unit) of the operation processing unit 10' side, and reference numeral 18 is a display unit for displaying the emission amount of the greenhouse effect gas calculated by the operation processing unit 10'.

That is, having the configuration of the example, in the operation processing unit 10', the measuring units 15, 16 are attached to the plural process effluents 3a, 3b installed in the enterprises, and are connected to the operation processing unit 10' through the network 6, so that a greenhouse effect gas emissions rights converting system 1' can be composed.

Therefore, by properly installing the measuring units 15, 16, ... according to the number of process effluents 3a, 3b, ..., each enterprise can easily check the emission index M of all greenhouse effect gas being emitted in real time. That is, the amount of emission can be accurately known as compared with the assigned emission framework, and the emissions rights can be purchased from an enterprise having a sufficient allowance, or an extra portion can be sold to an enterprise in need. The measuring units 15, 16, ... are simple in structure, and the manufacturing cost can be suppressed significantly.

As in this example, the operation processing unit 10' can be formed by using a desired conversion function formed on the network 6, and the configuration is not particularly limited. In this example, the server is installed in each enterprise, and the operation processing unit 10' is formed by using this server, but it may be also designed to execute by other computer on the network 6. For example, when the trade market 7 is designed to monitor the measured values by the measuring units 15, 16, it is possible to monitor if each enterprise is operating adequately from the viewpoint of global environment.

Anyway, each operation processing unit 10' processes by converting into the emission index of greenhouse effect gas by using conversion formulas 10a, 10b, ... and constants (conversion information) conforming to the standard determined by the organization of the IPCC 14, and the emission guidance of greenhouse effect gas can be calculated by a single conversion formula common to all, so that a fair emissions rights can be traded.

In addition, the emission guidance of greenhouse effect gas can be converted in a hierarchical structure. That is, summing up the total emission index calculated in each enterprise in each district, the total amount of greenhouse effect gas emitted in the district unit can be calculated, or the total emission index summed up in each district unit can be calculated on the national basis, and the total amount of greenhouse effect gases emitted in each nation can be compared. Still more, the emissions rights of greenhouse effect gas can be traded not only between enterprises, but also between nations, or communities.

In this example, the Internet 6 is used as general means of the network 6, but this point is not limited in the invention. For example, by using the intranet or limited communication network instead of the Internet, the security may be enhanced.

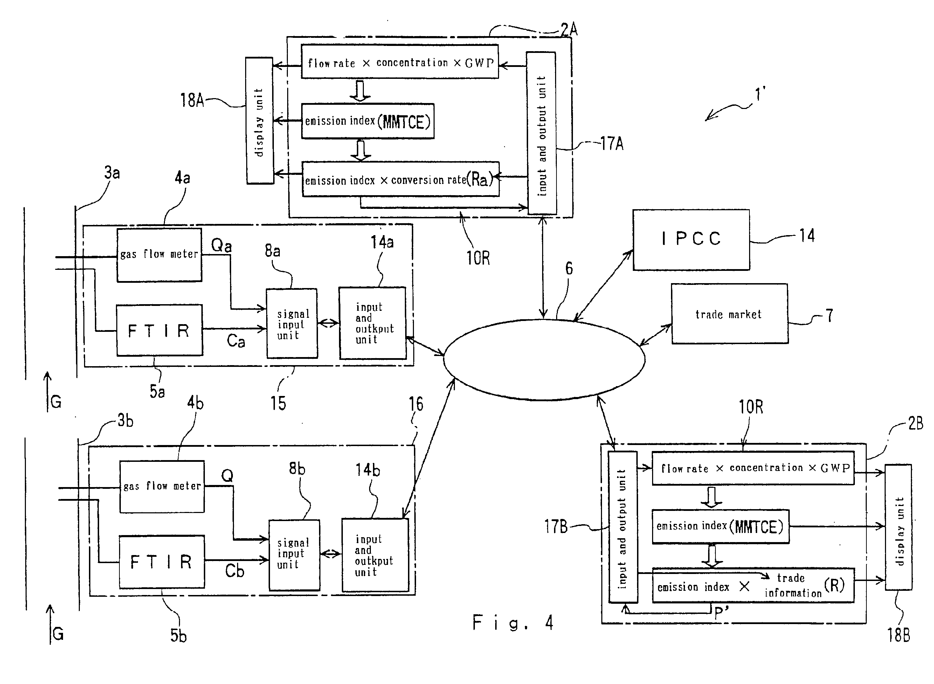

Fig. 4 is a diagram showing a modified example of the greenhouse effect gas emission index converting system 1'. In this example, too, same reference numerals as in Fig. 3 are same or corresponding parts. Reference numerals 2A, 2B, ... are operation processing units installed in enterprises. In this example, measuring units 15, 16 are installed in process effluents 3a, 3b controlled by the operation processing unit 2A. Reference numerals 17A, 17B, ... are input and output units provided in the operation processing units 2A, 2B, ..., and 18A, 18B, ... are display units.

As all operation processing unit 2A, 2B, ... execute a same conversion program 10R, values measured by all measuring units 15, 16, ... to be managed are taken in through the network 6 in calculation formulas shown in the above-mentioned formulas (6) to (8), so that the emission index M of greenhouse effect gas and emission expense P are determined.

In Fig. 4, keyboards 12a, 12b (see Fig. 3) are excluded from the measuring units 15, 16, and the structure is further simplified and the manufacturing cost is saved. Incidentally, the IPCC or other environmental management office may properly monitor the measuring units 15, 16 through the network 6 to check if the enterprises are conforming to the specified emission framework or not, and purchase of emissions rights may be advised to the enterprises running short of the emissions rights.

Besides, hierarchical calculation of emission amount of greenhouse effect gas and various modifications can be executed as explained in Fig. 3.

By using the greenhouse effect gas emission index converting system of the invention as described herein, the emission amount of greenhouse effect gas can be converted into a common and single transaction index, and can be converted into the emission index of greenhouse effect gas using the latest conversion formula and conversion coefficient.

Moreover, the total amount of the emitted greenhouse effect gases can be determined accurately from the measured values, and the emissions rights can be directly converted and issued as the amount of money of trade, so that an adequate emissions rights can be traded promptly.

**Reference Symbols**

| | |
|---|---|
| 1 | greenhouse effect gas emissions rights converting system |
| 1' | greenhouse effect gas emissions rights converting system |
| 2 | greenhouse effect gas emission index converter; operation processing unit |
| 3 | emission passage, process effluent |
| 3a | emission passage, process effluent |
| 3b | emission passage, process effluent |
| 4 | gas flow meter |
| 4a | gas flow meter |
| 4b | gas flow meter |
| 5 | gas analyzer |
| 5a | gas analyzer |
| 5b | gas analyzer |
| 6 | network, internet |
| 7 | trade market, conversion information suplly terminal |
| 8 | data input unit |
| 8a | signal input unit |
| 8b | signal input unit |
| 8A | signal input unit |
| 8B | key input unit |
| 9 | information input unit |
| 10 | operation processing unit |
| 10' | operation processing unit |
| 10a,b, ... | conversion information, conversion formula |
| 10A,B, ... | conversion information, conversion program |
| 11 | information output unit |
| 12 | keyboard |
| 13 | display, display unit |
| 14 | monitoring organization, IPCC |
| 15 | measuring unit |
| 16 | measuring unit |
| a | decomposition factor of abatement equipment/decontamination apparatus of a plant |
| $a_i$ | decomposition factor of abatement equipment/decontamination apparatus of a plant for i-th greenhouse effect gas |
| Bi | generation rate |
| C | consumption rate |
| Ci | gas consumption rate |
| $c_{CO_2}$ | concentration of $CO_2$ |
| $c_{CH_4}$ | concentration of $CH_4$ |
| $c_{N_2O}$ | concentration of $N_2O$ |

| | |
|---|---|
| $c_{HFC}$ | concentration of HFC |
| $c_{PFC}$ | concentration of PFC |
| $c_{SF_6}$ | concentration of $SF_6$ |
| b | decomposition factor of abatement equipment of a decomposition system |
| $d_i$ | decomposition factor of abatement equipment of a decomposition system for the i-th greenhouse effect gas |
| F | decomposition factor |
| FC | purchase weight of greenhouse effect gas |
| FCi | purchase weight of i-th greenhouse effect gas |
| FTIR | Fourier transform infrared ray analyzer |
| G | emission gas |
| GWP | global warming potential for gas |
| GWPi | 100-year value of global warming potential for gas of i-th greenhouse effect gas |
| $GWP_{FCi}$ | 100-year value of global warming potential for gas of i-th greenhouse effect gas |
| h | rate of unused gas |
| H | |
| IPCC | monitoring organization |
| M | emission index |
| MMTCE | carbon equivalent |
| p | process |
| P | emission expense |
| P' | trade amount |
| Pi | purchase amount |
| Q | flow rate |
| $Q_i$ | emission amount of i-th greenhouse effect gas |
| $Q_{total}$ | monetary value of the total emission of greenhouse effect gas |
| R | trade information |
| Ra | conversion rate |
| Rb | exchange rate |

**Claims**

1.  Greenhouse effect gas emission index converting system, comprising

    -   a data input unit (8) for entering various data for calculating the emission index (M) of greenhouse effect gas,
    -   an information input unit (9) for incorporating conversion information (10a, 10b, ...; 10A, 10B, ...) for calculating the emission index (M) of greenhouse effect gas by using various data,
    -   an operation processing unit (2, 10) for calculating the emission index (M) of greenhouse effect gas by calculating various data according to said conversion information (10a, 10b, ...; 10A, 10B, ...), and
    -   an information output unit (11) for issuing the calculated emission index (M).

2.  Greenhouse effect gas emission index converting system according to claim 1,

    -   wherein a part of the above-mentioned various data includes measured at least one concentration and/or flow rate of at least one greenhouse effect gas, and
    -   wherein the emission index (M) is calculated by integration of their multiplied products.

3.  Greenhouse effect gas emission index converting system according to anyone of the preceding claims, wherein at least one of incorporation of various data in the above-mentioned data input unit (8), incorporation of conversion information (10a, 10b, ...; 10A, 10B, ...) in the above-mentioned information input unit (9), or output of emission index (M) in the above-mentioned information output unit (11) is executed through a network (6).

4.  Greenhouse effect gas emission index converting system according to anyone of the preceding claims, wherein the above-mentioned conversion information (10a, 10b, ...; 10A, 10B, ...) involves at least one conversion formula (10a, 10b, ...) for calculating the emission index (M) of greenhouse effect gas determined by the monitoring organization (14, IPCC) for organizing the emission amount of greenhouse effect gas and/or the constants used in such conversion formula (10a, 10b, ...).

**5.** Greenhouse effect gas emission index converting system according to anyone of the preceding claims, wherein the above-mentioned conversion information (10a, 10b, ...) has the conversion rate for calculating the emission expense of greenhouse effect gas by multiplying with the emission index (M) of greenhouse effect gas, and the above-mentioned operation processing unit (2, 10) has a market reference function of entering the above-mentioned conversion rate, exchange rate and other trade information from the trade market of greenhouse effect gas through the above-mentioned information input unit (9).

**6.** Greenhouse effect gas emission index converting system according to anyone of the preceding claims, wherein the above-mentioned operation processing unit (2, 10) has a market disclosure function of outputting the trade amount by multiplying the trade information such as conversion rate and exchange rate with the emission index (M) of the above-mentioned greenhouse effect gas to the trade market (7) of greenhouse effect gas through the above-mentioned information output unit (11).

IPCC

trade market
(conversion information supply terminal)

14

6

7

3

4

gas flow meter

Q

gas analyzer

5

$C \begin{cases} C_{CO2} \\ C_{CH4} \\ C_{N20} \\ C_{HFC} \\ C_{PFC} \\ C_{SF6} \end{cases}$

signal input unit

8A

9

information input unit

information output unit

11

display unit

13

10a,10b,···
(10A,10B,···)
Ci, Bi, R(Ra,Rb,)

M, P
P'

G

key input unit

8B

FC
h
P

a
b

MMTCE
$= \dfrac{12}{44} \Sigma\{(1-h) \times FCi \times (1-Ci) \times GWPi$
$+ (1-h) \times Bi \times FCi \times GWPi\}$

10a
10A
10B
10C
10D

1

12

8

10

2

Fig. 1

Fig. 2

Fig. 3

trade market

IPCC

7

14

10'

10a

10A
10B
10C
10D

$$MMTCE = \frac{12}{44} \Sigma \{(1-h) \times FCi \times (1-Ci) \times GWPi + (1-h) \times Bi \times FCi \times GWPi\}$$

17

input and output unit

display unit

18

6

M, P
P'

1'

10a,10b,……
(10A,10B,……)
Ci, Bi
R(Ra,Rb,)

15

16

14a

input and
output unit

14b

input and
output unit

8a

input unit

8b

input unit

12a

gas flow meter

Qa

4a

5a

gas analyzer

Ca

12b

gas flow meter

Qb

4b

5b

gas analyzer

Cb

3a

3b

G

G

2A

flow rate × concentration × GWP

18A

emission index (MMTCE)

17A

emission index × conversion rate (Ra)

display unit

input and output unit

1'

3a    4a

gas flow meter    Qa    8a    14a

10R

FTIR    Ca    signal input unit    input and outkput unit

IPCC    14

5a    15

6    trade market    7

G

3b    4b

gas flow meter    Q    8b    14b

FTIR    Cb    signal input unit    input and outkput unit

16    10R    2B

17B    input and output unit

flow rate × concentration × GWP    display unit

emission index (MMTCE)

emission index ×    trade information    (R)

P'

18B

5b

G

F i g .  4